Europäisches Patentamt

European Patent Office

Office européen des brevets

(11) Publication number: **0 060 871**
**B1**

# EUROPEAN PATENT SPECIFICATION

(45) Date of publication of patent specification: **23.10.85**

(21) Application number: **81902716.0**

(22) Date of filing: **24.09.81**

(86) International application number:
**PCT/US81/01291**

(87) International publication number:
**WO 82/01192 15.04.82 Gazette 82/10**

(51) Int. Cl.⁴: **C 12 P 21/00, C 12 N 15/00,
G 01 N 33/53**

(54) **MONOCLONAL ANTIBODIES SPECIFIC FOR HUMAN HEMATOPOIETIC CELL SURFACE GLYCOPROTEINS.**

(30) Priority: **25.09.80 US 190728**

(43) Date of publication of application:
**29.09.82 Bulletin 82/39**

(45) Publication of the grant of the patent:
**23.10.85 Bulletin 85/43**

(84) Designated Contracting States:
**AT CH DE FR GB LI LU NL SE**

(56) References cited:
**US-A-4 172 124
US-A-4 196 265**

**JOURNAL OF IMMUNOLOGY, VOLUME 125,
NO. 5, ISSUED NOVEMBER 1980, T.W. LeBIEN
ET AL, MONOCLONAL ANTIBODY (TA-1)
REACTIVE WITH HUMAN T LYMPHOCYTES
AND MONOCYTES.**

The file contains technical information submitted after the application was filed and not included in this specification

(73) Proprietor: **THE SALK INSTITUTE FOR
BIOLOGICAL STUDIES
10010 North Torrey Pines Road
La Jolla California 92038 (US)**

(72) Inventor: **TROWBRIDGE, Ian S.
3105 Duccomun Avenue
San Diego, CA 92122 (US)**

(74) Representative: **Lawrence, Malcolm Graham
et al
BROOKES & MARTIN High Holborn House 52/54
High Holborn
London WC1V 6SE (GB)**

(56) References cited:
**NATURE, VOLUME 273, ISSUED 22 JUNE 1978,
M.M. TRUCCO ET AL, MONOCLONAL
ANTIBODIES AGAINST HUMAN LYMPHOCITE
ANTIGENS.
NATURE, VOLUME 286, ISSUED 28 AUGUST
1980, M.B. ANARY ET AL, HUMAN CELL-
SURFACE GLYCOPROTEIN WITH UNUSUAL
PROPERTIES.**

JOURNAL OF EXPERIMENTAL MEDICINE, VOLUME 148, NUMBER 1, ISSUED 1978, I.S. TROWBRIDGE, INTERSPECIES SPLEEN-MYCLOMA HYBRID PRODUCING MONOCLONAL ANTIBODIES AGAINST MOUSE LYMPHOCYTE SURFACE GLYCOPROTEIN t200.
NATURE, VOLUME 283, ISSUED 07 FEBRUARY 1980, J. RITZ ET AL, A MONOCLONAL ANTIBODY TO HUMAN ACUTE LYMPHOBLASTIC LEUKAEMIA ANTIGEN.
JOURNAL OF THE NATIONAL CANCER INSTITUTE, VOLUME 60, NUMBER 6, ISSUED JUNE 1978, J. MINOWADA ET AL, EXPRESSION OF AN ANTIGEN ASSOCIATED WITH ACUTE LYMPHOBLASTIC LEUKEMIA IN HUMAN LEUKEMIA-LYMPHOMA CELL LINES.

# Description

The present invention is directed to hybridoma cell lines which produce monoclonal antibodies and more particularly to monoclonal antibodies specific for glycoproteins widely distributed in human hematopoietic cells.

## Background of the invention

Antibodies have long been used in medical diagnosis, e.g., determining blood types, and in biological experimentation. The usefulness of antibodies, however, has been somewhat limited, as their complexity and diversity have made it very difficult to obtain homogeneous antibodies. Antibodies are complex protein or protein-based molecules which are produced by the immune systems of animals to protect the animal against foreign substances. Antibodies for medical use are generally obtained by injecting an animal with a foreign substance which will stimulate the animal's immune system and, most commonly, isolating an antibody fraction from the peripheral blood serum or from the ascitic fluid. The antibody fraction contains antibodies specific to the injected foreign substance as well as various other antibodies produced by the animal, and by known techniques, it may be possible to substantially isolate an antibody specific to the particular foreign substance. However, even when an antibody for a particular foreign substance is isolated, such antibody is actually a mixture of several antibodies which recognize various antigenic determinants of the foreign substance or related substances. While some individual antibody molecules may be highly specific, recognizing only a certain foreign substance or portion thereof, other antibody molecules may be less selective, recognizing not only the subject foreign substance but other substances as well. Since it is generally practically impossible to separate all related antibodies even the most carefully purified antibody fractions may react with more than one substance.

In recent years, techniques of producing monocloncal antibodies have been developed which make it possible to obtain homogeneous, highly specific antibodies. Generally, such antibodies are produced by immunizing an animal with a protein fraction or other foreign substance, obtaining antibody-producing cells from the animal, and fusing the antibody-producing cells with strains of myeloma cells, e.g. tumor cells, to produce hybridomas which are isolated and cultured as monoclones. The monoclonal hybridomas may either be cultured *in vitro* or may be grown as tumors in a host animal. Since each antibody-producing cell produces a single unique antibody, the monoclonal cultures of hybridomas each produce a homogeneous antibody which may be obtained either from the culture medium of hybridoma cultures grown *in vitro* or from the cells, ascitic fluid, or serum of a tumor-bearing host animal.

Not all of the hybridoma clones which result from fusing neoplastic cells with antibody-producing cells are specific for the desired foreign substance or antigen (a substance with which the antibody reacts) since many of the hybridomas will make antibodies which the inoculated animal has produced to react with other foreign substances. Even antibodies against the subject antigen will differ from clone to clone since antibodies produced by different cells may react with different antigenic determinants of the same molecule. From each clone, therefore, it is necessary to obtain the resulting antibody or the antibody-containing medium, serum or ascitic fluid and test its reactivity with the subject antigen and to test its specificity by determining with what other substances, if any, if recognizes. While the necessity of characterizing the antibody of each clone adds to the complexity of producing monoclonal antibodies, the wide variety of homogeneous antibodies which may be obtained gives investigators a number of very precise tools to map the structure and development of somatic cells.

The availability of homogeneous, highly specific monoclonal antibodies dramatically increases the value of antibodies as a diagnostic, experimental and therapeutic tool. Use of monoclonal antibodies for tumor and virus detection has been described in U.S. Patents Nos. 4,172,124 and 4,196,265.

Monoclonal antibodies are particularly suitable for studying the pathways and processes by which cells differentiate into different types of cells. The proteins and other macromolecules in cells which may be precisely detected by monoclonal antibodies may serve as important clues to the derivation of cell lines. This may be particularly important when undifferentiated cancer cells are detected in the body and where the treatment of the cancer is dependent on the type of tumor present. The morphology of undifferentiated lymphomas cells may closely resemble the morphology of carcinoma cells, but the accepted treatments of lymphomas and carcinomas differ substantially. Thus it is highly important that it be determined as quickly as possible whether a cancer cell line in a patient is a carcinoma or a lymphoma line. Where the identity of the tumor line is not apparent from its gross morphology, highly specific monoclonal antibodies may be used to classify cell lines by their molecular composition.

## Summary of the invention

Monoclonal antibodies are produced which are specific for cell surface glycoproteins, which are widely distributed on human hematopoietic cells, particularly human B and T lymphocytes lines, and which are generally absent from non-hematopoietic cells. Mice are inoculated with a line of human lymphocytes, and spleen cells or lymph node cells are obtained from the inoculated mice and fused with mice tumor cells. Monocultures of the fused cells are produced, and the antibodies obtained from the monoclones are tested for their reactivity with cell surface glycoproteins particular to nucleated hematopoietic cells to select the monoclones which produce antibodies reactive with the glycoproteins. The monoclonal antibodies may be particularly useful to distinguish undifferentiated carcinomas from undifferentiated lymphomas.

Detailed description of the preferred embodiments

Monoclonal antibodies are produced which are specific for a family of glycoproteins which have been designated T29/33 and which are widely distributed on the surface of human nucleated hematopoietic cells but which are absent from other human tissue cells. T29/33 glycoproteins are expressed on more than 95% of peripheral blood leukocytes, and a similar broad distribution is found in other lymphoid and hematopoietic tissues including spleen, thymus, bone marrow and tonsil. They do not appear in detectable amounts in erythrocytes, liver, brain or kidney tissue, nor a variety of other normal adult human tissues. T29/33 glycoproteins have been characterized as having molecular weights of between about 200,000 and about 220,000 as determined by their migration on SDS-polyacrylamide gel.

Similar hematopoietic cell surface glycoproteins are known in non-human species. A family of mouse glycoproteins designated T-200 has been identified, Trowbridge *J. Exp. Med. 148*:313, (1978). T-200 glycoproteins obtained from mouse cell lines ASL1 and BW5147, Hyman et al. *Cold Spring Harbor Symp. Quant. Biol. 41*:407 (1976), have been proteolysed and peptide mapped using techniques described in Omary et al. *J. Biol. Chem. 255*:1662 (1980).

A human glycoprotein designated B3/25 has been identified, (Trowbridge) Nature, Vol. 286, pp. 888—891, 28 August 1980. The B3/25 glycoprotein reported in the Trowbridge article exists under non-reducing conditions as a disulfide-bonded dimer which splits under reducing conditions to a monomeric form migrating on SDS-polyacrylamide gel with an apparent molecular weight of 100,000.

Lymphocytes are introduced into animals, to induce the production of antibodies reactive with proteins and glycoproteins, including T29/33 glycoproteins found on the cell surface of most nucleated hematopoietic cells. The animal chosen for inoculation is not critical, but it is preferred that the strain of animal be well characterized. Since various strains of murines, i.e. rats, mice, etc., are well characterized, and since various murine-derived neoplastic cells are also available as well-characterized cultures, mice are chosen for production of the antibodies hereindescribed, although it is to be understood that the invention is not limited to murine-derived monoclonal antibodies.

BALB/c mice are inoculated intraperitoneally with $2\times10^7$ cells from the normal human T leukemic line CCRF-CEM, Foley et al. *Cancer 18* 522, (1965), suspended in standard tissue culture media. After 2 weeks, the mice are inoculated with a booster of at least $10^6$ cells. Four days after the second inoculation, the mice are sacrificed and their spleens are taken. A spleen cell suspension is prepared in the manner taught by Gerhard et al., *Eur. J. Immunol. 5*, 720—725 (1975). The red blood cells are removed by lysing in 0.83% $NH_4Cl$ for 15 minutes at 4°C, and the resulting cell suspension is washed by one centri-fugation (800×g) in heat-inactivated horse serum and one centrifugation in protein-free Dulbecco's modified Eagles medium.

Since the antibody-producing cells obtained from the spleen do not independently reproduce, and thus cannot be cultured, they are fused with cells which may be independently cultured either *in vivo* or *in vitro* so that the genetic and metabolic processes of the fused hybridomas have characteristics of each of the parent cells, and it is intended that certain of the cells obtained will have the capabilities to independently reproduce and to produce the antibody of the antibody-producing parent cell. Some tumor cells, particularly myeloma cells, may be advantageously fused with antibody-producing cells to provide viable antibody-producing cultures of hybridomas. Although it is not necessary, it is preferred that the tumor cells and antibody-producing cells be derived from the same species to enhance the likelihood that the genetic and biochemical properties of the parent cells will be compatible and thus produce viable hybridomas. A number of myeloma cultures have been characterized, and herein, the mice-derived non-antibody producing myeloma cell line S194/ 5.XX0.BU.1, Trowbridge, *J. Exp. Med., 148*, 313— 323 (1978), samples of which are on deposit at the Salk Institute Cell Distribution Center, are used to produce the hybridomas. It is to be understood that other tumor lines, which include but are not limited to P3, Y3, SP2/0, MPC-11 and their derivatives, may also be used. It is advantageous to select a myeloma line which does not produce an antibody so that the resulting hybrid will only produce antibody chains of the parent spleen or lymph node cell.

The myeloma cells are maintained in Dulbecco's modified Eagle's medium supplemented with 10% horse serum. $10^7$ myeloma and $10^8$ cells obtained from the mice immunized with CCRF-CEM, are resuspended for fusion in a 40% solution (v/v) of polyethylene glycol 1500 according to the methods of Trowbridge *supra*. Cell hybrids are selected in hypoxanthine-amino-pterinthymidine (HAT) medium, all growth in HAT medium being indicative of successful hybridization of mouse spleen and mouse myeloma cells. Their production of antibodies against the lymphocytes used to inoculate the mice is tested by the antibody-binding assay described by Williams et al., *Cell 12*, 663 (1977). Hybrid cells are cloned by the method of limiting dilution in Falcon microtiter plates.

Clones of hybridomas may be grown *in vitro* according to known tissue culture techniques such as is described by Cotton et al., *Eur. J. Immunol. 3,* 136 (1973). Alternatively, hybridomas may be grown *in vivo* as tumors in a histocompatible animal or in athymic nude mice. The antibodies may be recovered from the *in vitro* culture medium or from the serum or ascitic fluid of the animal by means known in the art, e.g. Gerhard et al., *Proc. Natl. Acad. Sci., 75,* pp. 1510—1514 (1978). In some cases it may be advantageous to obtain the antibodies directly from the cells of the culture or tumor.

The specificity of the antibody from each clone for nucleated hematopoietic cells is examined by the methods of Williams *supra.,* and clones which produce antibody specific for nucleated hematopoietic cells are selected. When a useful hybridoma clone is produced, it is generally advantageous to reclone the cell line to avoid overgrowth of cultures with variant cells no longer producing antibody. Since the hybridoma contains some, but not all, of the genetic material of each parent cell, the full characteristics of the hybridoma are not known. Often a hybridoma clone, due to original genetic deficiency or subsequent chromosome loss, after several passages may lose its ability to reproduce and/or to produce the particular antibody. Accordingly, it is important, soon after the initial hybridization, that a hybridoma clone of interest is recloned to insure the availability of functioning strains of the antibody-producing hybridoma.

Example

Hybridoma cell lines are produced by mixing $10^8$ spleen cells obtained from BALB/c mice immunized with CCRF-CEM hematopoietic cells with $10^7$ S194/5.XX0Bu.1 cells. The cell mixture is centrifuged at 800×g, and the cells are resuspended for fusion in a 40% (v/v) solution of polyethylene glycol 1500 in modified Eagle's medium. Cell hybridomas are selected in HAT medium and cloned by the method of limiting dilution in microtiter plates. The culture media of the resulting monoclones are tested for immuno activity against hematopoietic cell lines and other human cell lines. Hybridoma cell lines are chosen for further culturing whose media exhibits immuno response to human hematopoietic cell lines and immuno response to other human cell lines. Monoclonal antibody fractions are obtained from the culture media of the selected hybridomas.

A monoclonal hybridoma cell line is produced and selected which produces a monoclonal antibody which reacts with the T29/33 glycoproteins in nucleated hematopoietic cells but does not react with other normal human cells including erythrocytes, liver, brain or kidney tissue. Because the cell line produces a monoclonal antibody specific for T29/33 glycoproteins, both the cell line and the monoclonal antibody produced thereby have been designated T29/33. The hybridoma cell line T29/33 is on deposit at the American Type Culture Collection under accession number CRL 8036. Certain derivatives of the T29/33 cell line also produce T29/33 monoclonal antibody.

The reactivity of the T29/33 monoclonal antibody with glycoproteins found on the surface of nucleated hematopoietic cells is further demonstrated by the molecular weight determination of the glycoproteins obtained by monoclonal antibody immunoprecipitation of hematopoietic cell lysates, Trowbridge et al., *Eur. J. Immunol. 6:*777, 1976. Lysates of [125]I-labeled CCRF-HSB2, a T-cell Lymphocytic leukemia cell line, Adams et al. *Exp. Cell Res. 62:*5 (1970), and CCRF-SB, a normal B-lymphocyte line, Foley et al. *Cancer 18:*522 (1965), are each precipitated with T29/33 monoclonal antibody. The precipitated glycoproteins from each cell line are electrophoresed on sodium dodecyl sulfate polyacrylamide gel, and two different molecular weight precipitates are obtained. The molecular weight of the T-cell T29/33 glycoprotein is about 200,000, and the molecular weight of the B-cell T29/33 glycoprotein is about 220,000. Both probably correspond to the high molecular weight glycoproteins in normal human T and B lymphocytes earlier reported, Andersson et al. *Int. J. Cancer 17:*40 (1976).

Further evidence of the hematopoietic cell specificity of T29/33 monoclonal antibody is the high degree of homology of the human glycoproteins with which the antibody reacts and mouse T200 hematopoietic cell surface glycoprotein. Human T leukemia cell lines CCRF-CEM and CCRF-HSB2 are both metabolically labeled with L-[35S] methionine, immunoprecipitated with T29/33 monoclonal antibody and proteolysed, Omary *supra.* Comparison of peptide maps produced from T200 ASL1 mouse cell glycoprotein proteolysates and T29/33 human T-cell glycoprotein proteolysates indicate that 17 labeled peptides of respective proteolysates are shared by the mouse and human glycoproteins, 15 peptides are unique to the mouse glycoprotein and 13 peptides are unique to the human glycoprotein.

Because T29/33 monoclonal antibody is generally reactive with hematopoietic cells and is nonreactive with other tissues, it serves as an important diagnostic tool in tumor biopsy for classifying cancer cell lines. The presence of T29/33 glycoproteins in a nonclassified cancer cell line, as determined by immunoprecipitation, radioimmunoassay, autoradiography, immunofluorescence or other known immuno techniques utilizing T29/33 monoclonal antibody, is a strong indication that the cancer cell line is a lymphoma rather than a carcinoma.

Modifications obvious to one with ordinary skill in the art may be made without departing from the scope of the present invention. For example, antibody production may be induced in the host animal by inoculating the animal with other human hematopoietic cell lines containing a T29/33 glycoprotein or with cell membrane fragments or cell-membrane-derived material rather than with complete hematopoietic cells.

Various features of the invention are set forth in the following claims.

**Claims for the Contracting States CH DE FR LI LU NL SE GB**

1. A hybridoma cell line produced by the fusion of an antibody-producing animal cell and a neoplastic cell, said animal cell being one which produces an antibody specific for a family of glycoproteins characterized by a molecular weight of between about 200,000 and about 220,000 and by their presence on human nucleated hematopoietic cells and their absence from other normal human tissue cells, the antibody-producing capability of the animal cell surviving fusion so that the hybridoma itself produces the antibody specific for said glycoproteins.

2. A cell line according to claim 1 wherein said antibody-producing cells are BALB/c mouse spleen cells.

3. A cell line according to claim 1 wherein said neoplastic cell is of the non-antibody-producing myeloma cell line, S194/5.XX0.BU.1.

4. A cell line according to any preceding claim in combination with a standard growth medium.

5. Monoclonal antibodies specific for a family of glycoproteins characterized by their presence on human nucleated hematopoietic cells and their absence from other normal human tissue cells, said glycoproteins having molecular weights of between about 200,000 and about 220,000.

6. Monoclonal antibodies according to claim 5 from a hybridoma cell line designated T29/33 (ATCC CRL8036) and its derivatives.

7. A method for distinguishing between undifferentiated lymphomas and undifferentiated carcinomas comprising inoculating an animal with either human nucleated hematopoietic cells or material derived therefrom, obtaining antibody-producing animal cells from said animal, fusing said antibody-producing cells and neoplastic cells to produce hybridomas, selecting a hybridoma that produces an antibody specific to cell surface glycoproteins present on human nucleated hematopoietic cells and absent from other normal cells, said glycoproteins having molecular weight of between about 200,000 and about 220,000 producing clones of said selected hybridoma, collecting the antibody produced thereby, and testing an uncharacterized cancer cell line for reactivity with said antibody.

8. A method according to claim 7 wherein said human hematopoietic cells are CCRF-CEM cells.

9. A method according to claim 7 wherein said neoplastic cells are of the nonproducer myeloma cell line, S194/5.XXO.BU.1.

**Claims for Contracting State AT**

1. A hybridoma cell line produced by the fusion of an antibody-producing animal cell and a neoplastic cell, said animal cell being one which produces an antibody specific for a family of glycoproteins characterized by a molecular weight of between about 200,000 and about 220,000 and by their presence on human nucleated hematopoietic cells and their absence from other normal human tissue cells, the antibody-producing capability of the animal cell surviving fusion so that the hybridoma itself produces the antibody specific for said glycoproteins.

2. A cell line according to claim 1 wherein said antibody-producing cells are BALB/c mouse spleen cells.

3. A cell line according to claim 1 wherein said neoplastic cell is of the non-antibody-producing myeloma cell line, S194/5.XXO.BU.1.

4. A cell line according to any preceding claim in combination with a standard growth medium.

5. A method of producing monoclonal antibodies specific for a family of glycoproteins characterized by their presence on human nucleated hematopoietic cells and their absence from other normal human tissue cells, said glycoproteins having molecular weights of between about 200,000 and about 220,000, the method comprising inoculating an animal with material containing said glycoproteins; obtaining antibody-producing cells from said animal; fusing said antibody-producing cells with neoplastic cells to produce hybridomas; selecting hybridomas which produce antibodies reactive with said family of glycoproteins; culturing said selected hybridomas as monoclones and collecting the monoclonal antibodies produced thereby.

6. A method according to Claim 5 wherein said glycoprotein-containing material is human hematopoietic cells or human hematopoietic cell membrane material.

7. A method for distinguishing between undifferentiated lymphomas and undifferentiated carcinomas comprising inoculating an animal with either human nucleated hematopoietic cells or material derived therefrom, obtaining antibody-producing animal cells from said animal, fusing said antibody-producing cells and neoplastic cells to produce hybridomas, selecting a hybridoma that produces an antibody specific to cell surface glycoproteins present on human nucleated hematopoietic cells and absent from other normal cells, said glycoproteins having molecular weight of between about 200,000 and about 220,000, producing clones of said selected hybridoma, collecting the antibody produced thereby, and testing an uncharacterized cancer cell line for reactivity with said antibody.

8. A method according to claim 7 wherein said human hematopoietic cells are CCRF-CEM cells.

9. A method according to claim 7 wherein said neoplastic cells are of the nonproducer myeloma cell line, S194/5.XXO.BU.1.

**Patentansprüche für die Vertragsstaaten CH DE FR LI LU NL SE GB**

1. Durch Verschmelzen einer Antikörper produzierenden Tierzelle und einer neoplastischen Zelle gebildete Hybridomzellart, wobei die Tierzelle einen für eine Familie von durch ein Molekulargewicht zwischen etwa 200 000 und etwa 220 000 und durch ihre Anwesenheit auf menschlichen kernhaltigen blutbildenden Zellen und durch ihre Abwesenheit von anderen menschlichen Gewebezellen gekennzeichneten Glykoproteinen spezifischen Antikörper produziert, und wobei das Antikörperproduktionsvermögen der Tierzelle das Verschmelzen so übersteht, daß das Hybridom selbst den für die besagten Glykoproteine spezifischen Antikörper produziert.

2. Zellart nach Anspruch 1, worin die Antikörper produzierenden Zellen Mäusemilzzellen BALB/c sind.

3. Zellart nach Anspruch 1, worin die neoplastische Zelle eine der nicht Antikörper produzierenden Myelomzellart, S194/5.XXO.BU.1., ist.

4. Zellart nach einem der vorhergehenden Ansprüche in Verbindung mit einem Standardwachstumsmedium.

5. Für eine Familie von durch ihre Anwesenheit auf menschlichen kernhaltigen blutbildenden Zellen und durch ihre Abwesenheit von anderen normalen menschlichen Gewebezellen gekennzeichneten Glykoproteinen spezifisch monoklonale Antikörper, wobei die Glykoproteine ein Molekulargewicht zwischen etwa 200 000 und etwa 220 000 haben.

6. Monoklonale Antikörper nach Anspruch 5 von einer Hybridomzellart mit der Bezeichnung T29/33 (ATCC CRL8036) und ihre Abkömmlinge.

7. Verfahren zur Unterscheidung zwischen undifferenzierten Lymphomen und undifferenzierten Karzinomen, welches Beimpfen eines Tieres entweder mit menschlichen kernhaltigen blutbildenden Zellen oder davon abgeleitetem Material, Gewinnung von Antikörper produzierenden Tierzellen aus dem Tier, Verschmelzen der Antikörper produzierenden Zellen und neoplastischen Zellen zu Hybridomen, Auswählen eines Hybridoms, welches einen für auf menschlichen kernhaltigen Zellen anwesende und in anderen normalen Zellen abwesende Zelloberflächenglykoproteine spezifischen Antikörper produziert, wobei die Glykoproteine ein Molekulargewicht zwischen etwa 200 000 und etwa 220 000 haben, Produzieren von Klonen von dem ausgewählten Hybridom, Sammeln des so gebildeten Antikörpers und Untersuchung einer nicht gekennzeichneten Krebszellenart auf Reaktivität mit dem Antikörper umfaßt.

8. Verfahren nach Anspruch 7, worin die menschlichen blutbildenden Zellen CCRF-CEM-Zellen sind.

9. Verfahren nach Anspruch 7, worin die neoplastischen Zellen von der nicht produzierenden Myelomzellart, S194/5.XXO.BU.1., sind.

**Patentansprüche für den Vertragsstaat AT**

1. Durch Verschmelzen einer Antikörper produzierenden Tierzelle und einer neoplastischen Zelle gebildete Hybridomzellart, wobei die Tierzelle einen für eine Familie von durch ein Molekulargewicht zwischen etwa 200 000 und etwa 220 000 und durch ihre Anwesenheit auf menschlichen kernhaltigen blutbildenden Zellen und durch ihre Abwesenheit von anderen menschlichen Gewebezellen gekennzeichneten Glykoproteinen spezifischen Antikörper produziert, und wobei das Antikörperproduktionsvermögen der Tierzelle das Verschmelzen so übersteht, daß das Hybridom selbst den für die besagten Glykoproteine spezifischen Antikörper produziert.

2. Zellart nach Anspruch 1, worin die Antikörper produzierenden Zellen Mäusemilzzellen BALB/c sind.

3. Zellart nach Anspruch 1, worin die neoplastische Zelle eine der nicht Antikörper produzierenden Myelomzellart, S194/5.XXO.BU.1., ist.

4. Zellart nach einem der vorhergehenden Ansprüche in Verbindung mit einem Standardwachstumsmedium.

5. Verfahren zur Herstellung von für eine Familie von durch ihre Anwesenheit auf menschlichen kernhaltigen blutbildenden Zellen und durch ihre Abwesenheit von anderen normalen menschlichen Gewebezellen gekennzeichneten Glykoproteinen spezifischen monoklonalen Antikörpern, wobei die Glykoproteine ein Molekulargewicht zwischen etwa 200 000 und etwa 220 000 haben, wobei das Verfahren Beimpfen eines Tieres mit die Glykoproteine enthaltendem Material, Gewinnung von Antikörper produzierenden Zellen aus dem Tier, Verschmelzen der Antikörper produzierenden Zellen mit neoplastischen Zellen zu Hybridomen, Auswählen von Hybridomen, welche mit der Familie von Glykoproteinen reaktive Antikörper produzieren, Kultivieren der ausgewählten Hybridome als Monoklone und Sammeln der dadurch gebildeten Monoklone umfaßt.

6. Verfahren nach Anspruch 5, worin das Glykoprotein enthaltende Material menschliche blutbildende Zellen oder menschliches blutbildendes Zellmembranmaterial ist.

7. Verfahren zur Unterscheidung zwischen undifferenzierten Lymphomen und undifferenzierten Karzinomen, welches Beimpfen eines Tieres entweder mit menschlichen kernhaltigen blutbildenden Zellen oder davon abgeleitetem Material, Gewinnung von Antikörper produzierenden Tierzellen aus dem Tier, Verschmelzen der Antikörper produzierenden Zellen und neoplastischen Zellen zu Hybridomen, Auswählen eines Hybridoms, welches einen für auf menschlichen kernhaltigen Zellen anwesende und in anderen normalen Zellen abwesende Zelloberflächenglykoproteine spezifischen Antikörper produziert, wobei die Glykoproteine ein Molekulargewicht zwischen etwa 200 000 und etwa 220 000 haben, Produzieren von Klonen von dem ausgewählten Hybridom, Sammeln des so gebildeten Antikörpers und Untersuchung einer nicht gekennzeichneten Krebszellenart auf Reaktivität mit dem Antikörper umfaßt.

8. Verfahren nach Anspruch 7, worin die menschlichen blutbildenden Zellen CCRF-CEM-Zellen sind.

9. Verfahren nach Anspruch 7, worin die neoplastischen Zellen von der nicht produzierenden Myelomzellart, S194/5.XXO.BU.1., sind.

**Revendications pour les Etats Contractants CH DE FR LI LU NL SE GB**

1. Une lignée de cellules d'hybridome produite par fusion d'une cellule d'animal productrice d'anticorps et d'une cellule néoplastique, ladite cellule animale étant une cellule qui produit un anticorps spécifique d'une famille de glycoprotéines caractérisées par un poids moléculaire entre environ 200 000 et environ 220 000 et par leur présence sur les cellules hématopoïétiques nucléées humaines et leur absence des autres cellules des tissus humains normaux et la capacité de produire un anticorps de la cellule animale survivant à la fusion si bien que l'hybridome lui-même produit l'anticorps spécifique desdites glycoprotéines.

2. Une lignée de cellules selon la revendication 1 dans laquelle lesdites cellules produisant un anticorps sont des cellules spléniques de souris BALB/c.

3. Une lignée de cellules selon la revendication 1 dans laquelle ladite cellule néoplastique est une cellule de la lignée de cellules de myélome ne produisant pas d'anticorps S194/5.XX0.BU.1.

4. Une lignée de cellules selon l'une quelconque des revendications précédentes en combinaison avec un milieu de culture standard.

5. Anticorps monoclonaux spécifiques d'une famille de glycoprotéines caractérisées par leur présence sur les cellules hématopoïétiques nucléées humaines et leur absence des cellules des autres tissus humains normaux, lesdites glycoprotéines ayant des poids moléculaires entre environ 200 000 et 220 000.

6. Anticorps monoclonaux selon la revendication 5 provenant d'une lignée de cellules d'hybridome appelées T29/33 (ATCC CRL8036) et de ses dérivés.

7. Un procédé pour distinguer les lymphomes indifférenciés des carcinomes indifférenciés comprenant l'inoculation d'un animal avec soit des cellules hématopoïétiques nucléées humaines, soit un matériau qui en dérive, l'obtention de cellules animales productrices d'anticorps à partir dudit animal, la fusion desdites cellules productrices d'anticorps et de cellules néoplastiques pour produire des hybridomes, la sélection d'un hybridome produisant un anticorps spécifique des glycoprotéines de surface cellulaire présentes sur les cellules hématopoïétiques nucléées humaines et absentes des autres cellules normales, lesdites glycoprotéines ayant un poids moléculaire entre environ 200 000 et environ 220 000, la production de clones dudit hybridome choisi, la récolte de l'anticorps ainsi produit et la détermination de la réactivité avec ledit anticorps d'une lignée de cellules cancéreuses non caractérisées.

8. Un procédé selon la revendication 7 dans lequel lesdites cellules hématopoïétiques humaines sont des cellules CCRF-CEM.

9. Un procédé selon la revendication 7 dans lequel lesdites cellules néoplastiques sont des cellules de la lignée de cellules de myélome non productrices S194/5.XX0.BU.1.

**Revendications pour l'Etat Contractant AT**

1. Une lignée de cellules d'hybridome produite par fusion d'une cellule d'animal productrice d'anticorps et d'une cellule néoplastique, ladite cellule animale étant une cellule qui produit un anticorps spécifique d'une famille de glycoprotéines caractérisées par un poids moléculaire entre environ 200 000 et environ 220 000 et par leur présence sur les cellules hématopoïétiques nucléées humaines et leur absence des autres cellules des tissus humains normaux et la capacité de produire un anticorps de la cellule animale survivant à la fusion si bien que l'hybridome lui-même produit l'anticorps spécifique desdites glycoprotéines.

2. Une lignée de cellules selon la revendication 1 dans laquelle lesdites cellules produisant un anticorps sont des cellules spléniques de souris BALB/c.

3. Une lignée de clelules selon la revendication 1 dans laquelle ladite cellule néoplastique est une cellule de la lignée de cellules de myélome ne produisant pas d'anticorps S194/5.XX0.BU.1.

4. Une lignée de cellules selon l'une quelconque des revendications précédentes en combinaison avec un milieu de culture standard.

5. Un procédé de production d'anticorps monoclonaux spécifiques d'une famille de glycoprotéines caractérisées par leur présence sur les cellules hématopoïétiques nucléées humaines et leur absence des autres cellules des tissus humains normaux, lesdites glycoprotéines ayant des poids moléculaires entre environ 200 000 et environ 220 000, le procédé comprenant l'inoculation d'un animal avec un matériau contenant lesdites glycoprotéines; l'obtention de cellules productrices d'anticorps à partir dudit animal, la fusion desdites cellules productrices d'anticorps avec des cellules néoplastiques pour produire des hybridomes; la sélection des hybridomes qui produisent des anticorps réagissant avec ladite famille de glycoprotéines; la culture desdits hybridomes choisis sous forme de monoclones et la récolte des anticorps monoclonaux ainsi produits.

6. Un procédé selon la revendication 5 dans lequel ledit matériau contenant des glycoprotéines est constitué de cellules hématopoïétiques humaines ou d'un matériau de membranes cellulaires hématopoïétiques humaines.

7. Un procédé pour distinguer les lymphomes indifférenciés des carcinomes indifférenciés comprenant l'inoculation d'un animal avec soit des cellules hématopoïétiques nucléées humaines, soit un matériau qui en dérive, l'obtention de cellules animales productrices d'anticorps à partir dudit animal, la fusion desdites cellules productrices d'anticorps et de cellules néoplastiques pour produire des hybridomes, la sélection d'un hybridome produisant un anticorps spécifique des glycoprotéines de surface cellulaire présentes sur les cellules hématopoïétiques nucléées humaines et absentes des autres cellules normales, lesdites glycoprotéines ayant un poids moléculaire entre 200 000 et environ 220 000, la production de clones dudit hybridome choisi, la récolte de l'anticorps ainsi produit et la détermination de la réactivité avec ledit anticorps d'une lignée de cellules cancéreuses non caractérisées.

8. Un procédé selon la revendication 7 dans lequel lesdites cellules hématopoïétiques humaines sont des cellules CCRF-CEM.

9. Un procédé selon la revendication 7 dans lequel lesdites cellules néoplastiques sont des cellules de la lignée de cellules de myélome non productrices S194/5.XX0.BU.1.